# EUROPÄISCHE PATENTSCHRIFT

(11) **EP 2 739 356 B1**
(45) Veröffentlichungstag und Bekanntmachung des Hinweises auf die Patenterteilung: **07.10.2015**
(21) Anmeldenummer: 12751448.7
(22) Anmeldetag: 03.08.2012
(51) Int. Cl.: A61N 5/10

(54) **VERBESSERTER ENERGIEMODULATOR**
IMPROVED ENERGY MODULATOR
MODULATEUR D'ÉNERGIE AMÉLIORÉ

(30) Priorität: 04.08.2011 DE 102011109380
(43) Veröffentlichungstag der Anmeldung: 11.06.2014
(73) Patentinhaber: GSI Helmholtzzentrum für Schwerionenforschung GmbH, 64291 Darmstadt (DE)
(72) Erfinder: SAITO, Nami, 64291 Darmstadt (DE); BERT, Christoph, 91080 Uttenreuth (DE); RIETZEL, Eike, 64331 Weiterstadt (DE)
(74) Vertreter: Blumbach Zinngrebe
(86) Internationale Anmeldenummer: PCT/EP2012/003321
(87) Internationale Veröffentlichungsnummer: WO 2013/017285

(56) Entgegenhaltungen:
- EP-A1- 0 817 210
- EP-A1- 1 371 390
- DE-A1- 19 907 098
- US-A- 5 440 133
- US-A1- 2004 200 983
- US-A1- 2008 240 352

## Beschreibung

Die Erfindung betrifft eine Teilchenenergieanpassvorrichtung zur variablen Veränderung der Energie der Teilchen eines durch die Teilchenenergieanpassvorrichtung hindurch tretenden Teilchenstrahls, die zumindest eine variable Energievariationseinrichtung aufweist. Weiterhin betrifft die Erfindung eine Stellwertkorrektureinrichtung für eine Teilchenenergieanpassvorrichtung zur variablen Veränderung der Energie der Teilchen eines durch die Teilchenenergieanpassvorrichtung hindurch tretenden Teilchenstrahls. Schließlich betrifft die Erfindung auch ein Verfahren zur Bestimmung von Korrekturwerten, insbesondere zur Verwendung für eine Teilchenenergieanpassvorrichtung und/oder für eine Stellwertkorrektureinrichtung vom vorab beschriebenen Typ.

Zwischenzeitlich wird in den unterschiedlichsten Gebieten der Technik eine Bestrahlung von Gegenständen durchgeführt. Je nach konkretem Einsatzerfordernis werden hierbei verschiedenartige Bestrahlungsverfahren sowie unterschiedliche Arten von Strahlung genutzt. So ist es in manchen Gebieten der Technik erforderlich, Gegenstände flächig bzw. räumlich und hierbei möglichst gleichmäßig zu bestrahlen. Dies ist beispielsweise der Fall, wenn Materialien gehärtet oder auf sonstige Weise verändert werden sollen. Auch ist es beispielsweise zwischenzeitlich auf dem Gebiet der Lebensmitteltechnik üblich geworden, Lebensmittel unter Verwendung bestimmter Strahlungsarten haltbar zu machen.

In anderen Gebieten der Technik ist es wiederum erforderlich, bestimmte Teilbereiche des zu bestrahlenden Gegenstands mit einer bestimmten vorab definierten, typischerweise besonders hohen Dosis zu bestrahlen. Die übrigen Teile des Gegenstands sollen dagegen in der Regel nicht bzw. so wenig wie möglich bestrahlt werden. Ein Beispiel hierfür ist die Strukturierung von Mikroprozessoren oder sonstigen Mikrostrukturen bzw. Nanostrukturen unter Verwendung von elektromagnetischer Strahlung (zum Teil bis in den Röntgenbereich hinein) sowie von bildgebenden Masken.

Die in den jeweiligen Strukturen zu applizierende Dosis kann dabei nicht nur in zwei Dimensionen strukturiert sein, sondern vielmehr auch in allen drei Raumrichtungen strukturiert sein. Durch eine dreidimensionale Strukturierung ist es beispielsweise möglich, einen Volumenbereich, der im Inneren eines zu bestrahlenden Körpers liegt, direkt und unmittelbar zu bestrahlen, ohne dass es erforderlich ist, den Körper (insbesondere dessen Außenhülle) zu beschädigen bzw. öffnen zu müssen.

Bei dem zu bestrahlenden Körper (bzw. einem im Inneren des zu bestrahlenden Körpers liegenden, zu bestrahlenden Volumenbereich) kann es sich im Übrigen nicht nur um einen statischen Körper bzw. um einen unbewegten Körper handeln. Vielmehr tritt in der Praxis oftmals das Problem auf, dass sich der zu bestrahlende Körper bzw. sich Teile des zu bestrahlenden Körpers (insbesondere ein zu bestrahlender Zielvolumenbereich) bewegen. Diese Bewegung kann nicht nur nach Art eines in sich starren Körpers, der gegenüber einem äußeren Koordinatensystem bewegt wird, vorliegen. Vielmehr ist es auch möglich, dass es zu einer Verschiebung unterschiedlicher Bereiche des zu bestrahlenden Körpers gegeneinander kommt. Dies betrifft die nicht notwendigerweise nur Translationsbewegungen. Vielmehr sind auch andere Arten von Veränderungen, wie insbesondere Rotationsbewegungen und Dichteänderungen denkbar.

Um derartige (zum Teil in sich) bewegte Körper bestrahlen zu können, werden sogenannte vierdimensionale Bestrahlungsverfahren verwendet. Dabei handelt es sich schlussendlich um dreidimensionale Bestrahlungsverfahren, die eine zeitliche Variation aufweisen (mit der Zeit als vierter Dimension). Beispiele für derartige Materialbestrahlungsverfahren finden sich im Bereich der Materialwissenschaften, beispielsweise bei der Herstellung hochintegrierter Bauteile (insbesondere von Mikroprozessoren und/oder von Speicherchips), sowie bei der Herstellung von mikrostrukturierten und nanostrukturierten Mechaniken.

Ein weiteres Gebiet der Technik, bei dem mittlerweile derartige, dreidimensionale bzw. vierdimensionale Bestrahlungsverfahren eingesetzt werden, liegt im Bereich der Medizintechnik vor. Auch hier ist es in der Regel erforderlich, bestimmte Volumenbereiche innerhalb eines Körpers (wie beispielsweise Tumore) mit einer möglichst hohen Dosis zu beaufschlagen, wohingegen das umgebende (gesunde) Gewebe möglichst wenig bzw. vorzugsweise im Wesentlichen nicht mit einer Dosis belastet werden sollte. Dies gilt im besonderen Maße, wenn es sich bei dem umgebenden Gewebe um ein sogenanntes kritisches Gewebe handelt, wie beispielsweise um empfindliche Organe (fachsprachlich als OAR für englisch: "Organ At Risk" bezeichnet) handelt. Hierbei kann es sich beispielsweise um das Rückenmark, um Hauptblutgefäße oder Nervenknoten handeln. Gerade bei der Bestrahlung von bewegten Zielvolumina treten vielfältige, zum Teil noch nicht bzw. nicht befriedigend gelöste Probleme auf.

Grundsätzlich gibt es eine große Anzahl an Lösungsmöglichkeiten. Speziell für die Verwendung mit Scanning-Verfahren werden insbesondere drei spezielle Herangehensweisen diskutiert. Hierbei handelt es sich um sogenannte Rescanning-Verfahren, Gating-Verfahren sowie Tracking-Verfahren.

Bei Rescanning-Verfahren wird der zu bestrahlende Körper mit einer großen Anzahl von Bestrahlungsvorgängen belegt. Bei einem zyklisch wiederkehrenden Bewegungsmuster des bewegten Körpers (bzw. des zu bestrahlenden Zielgebiets) erfolgt dadurch im statistischen Mittel eine ausreichend hohe Bestrahlung des Zielvolumens.

Bei Gating-Verfahren erfolgt eine aktive Bestrahlung des Zielkörpers nur dann, wenn sich der zu bestrahlende Volumenbereich in einer relativ eng begrenzten Bewegungsphase befindet. Zu anderen Zeitpunkten erfolgt dagegen keine Bestrahlung.

Als besonders aussichtsreich werden derzeit vor allem Tracking-Verfahren angesehen. Hierbei wird der Bereich, in dem die Bestrahlung schlussendlich einwirkt (zum Beispiel die Zone des Bragg-Peaks) korrespondierend zur Bewegung des zu bestrahlenden Volumenbereichs des Zielkörpers bewegt.

Allen drei Verfahren ist gemeinsam, dass der Teilchenstrahl (genauer: das hauptsächliche Wirkgebiet der Teilchen) in allen drei Raumdimensionen abgefahren (durchgescannt) werden muss. Um ein Scannen in z-Richtung (Richtung im Wesentlichen parallel zum Teilchenstrahl) zu realisieren ist es dabei erforderlich, die Energie der Teilchen zu variieren.

Eine Möglichkeit dies zu realisieren besteht darin, dass der Teilchenbeschleuniger selbst derart variierend angesteuert wird, dass dieser Teilchen mit unterschiedlicher Energie ausstößt. Problematisch ist hierbei, dass die Variation der Teilchenenergie hierbei nur relativ langsam erfolgen kann. Bei Synchotrons beispielsweise ist es bislang bestenfalls möglich, die Teilchenenergie von einem Extraktionszyklus zum nächsten zu variieren. Hier kommt es somit zu Energiestellzeiten im Bereich von ca. 10 s. Insbesondere für Tracking-Verfahren sind derartige Stellzeiten zu lang und somit nicht geeignet. Aber auch für Rescanning-Verfahren und Gating-Verfahren kommt es bei derartig langen Stellzeiten zu einem unnötigen Verlust an Strahlzeit in einem signifikanten Ausmaß.

Als Lösungsmöglichkeit wurden bereits passive Energiemodulatoren vorgeschlagen. Bei diesen läuft der Teilchenstrahl durch ein einergieabsorbierendes Medium hindurch. Durch einen geeigneten Stellmechanismus kann das Medium in Bezug auf seine Dicke (so wie sie vom Teilchenstrahl "wahrgenommen" wird) hin verändert werden, sodass der Teilchenstrahl eine unterschiedliche Länge durch das energieabsorbierende Material hindurch treten muss. Dadurch wird die Energie der hindurch tretenden Teilchen dementsprechend verändert. Als derartige Absorbersysteme sind beispielsweise keilartige bzw. doppelkeilartige Energieabsorbersysteme bekannt. Weiterhin wurden schnell bewegliche Wassersäulen sowie drehbare Modulatorräder vorgeschlagen. Auch bei diesen wird schlussendlich die Länge, die die Teilchen durch das entsprechende Modulatormaterial hindurch treten müssen, verändert.

Die Patentanmeldung US 2004/200983 A1 offenbart eine solche variable Energievariationseinrichtung wobei die Einstelleinrichtung anhand von Kalibrationsdaten korregiert werden kann.

Obgleich derartige Modulatorsysteme grundsätzlich für eine schnelle Energiemodulation geeignet sind, weisen diese nach wie vor Nachteile auf. Beispielsweise hat sich gezeigt, dass sich zwischen einer "angesteuerten" Energiedämpfung (also dem Eingangswert des Stellsignals) und der tatsächlichen Energiedämpfung durch das Modulatorsystem zum Teil beachtliche Diskrepanzen ergeben können. Dies führt zu entsprechenden Ungenauigkeiten beim Bearbeitungsvorgang bzw. Behandlungsvorgang, was entsprechend nachteilig ist.

Die Aufgabe der Erfindung besteht somit darin, eine gegenüber dem Stand der Technik verbesserte Teilchenenergieanpassvorrichtung, eine gegenüber dem Stand der Technik verbesserte Stellwertkorrektureinrichtung sowie ein gegenüber dem Stand der Technik verbessertes Verfahren zur Bestimmung von Korrekturwerten vorzuschlagen.

### Die Erfindung löst diese Aufgabe.

Es wird vorgeschlagen, eine Teilchenenergieanpassvorrichtung zur variablen Veränderung der Energie der Teilchen eines durch die Teilchenenergieanpassvorrichtung hindurch tretenden Teilchenstrahls, welche zumindest eine variable Energievariationseinrichtung aufweist, derart auszubilden, dass zumindest eine Stellwertkorrektureinrichtung zur Korrektur eines einer Teilchenenergieanpassvorrichtung zu geführten Stellwerts vorhanden ist, wobei die Stellwertkorrektureinrichtung derart ausgebildet und eingerichtet ist, dass die der Teilchenenergieanpassvorrichtung zugeführten Stellwerte zumindest zeitweise und/oder zumindest teilweise unter Verwendung von Kalibrationsdaten korrigiert werden. Die Erfinder haben festgestellt, dass bei Teilchenenergieanpassvorrichtungen eine größere Anzahl von potentiellen Fehlerquellen vorhanden ist. So können beispielsweise Materialungenauigkeiten eine lokal unterschiedliche Dämpfungswirkung entfalten, obgleich die Länge der durchstrahlten Materie "passend" ist. Ebenfalls ist es möglich, dass es aufgrund von Fertigungstoleranzen (insbesondere bei keilartigen Absorptionssystemen) zu unbeabsichtigten Dickenschwankungen des Absorptionsmaterials kommt. Eine weitere potentielle Fehlerquelle liegt in den Stellantrieben sowie in der Montage der Teilchenenergieanpassvorrichtung im Bereich des Teilchenstrahls selbst. Verblüffenderweise ist die Mehrzahl der potentiellen Fehlerquellen systematischer Natur. Dadurch wird es verblüffenderweise möglich, geeignete Kalibrationsdaten zu ermitteln und unter Verwendung dieser Kalibrationsdaten das "Endergebnis" der Energieanpassung der Teilchen zu verbessern. Dies kann wiederum in einer Verbesserung der Qualität der Bestrahlung resultieren. Grundsätzlich ist es möglich, die Kalibrationsdaten auf beliebige Weise zu erhalten. Als bevorzugt hat es sich jedoch herausgestellt, wenn die Kalibrationsdaten experimentell (also durch Vermessung der Teilchenenergieanpassvorrichtung) erzielt werden. Hierdurch können in der Regel besonders exakte Kalibrationsdaten ermittelt werden. Darüber hinaus können derartige, durch Messung ermittelte Kalibrationsdaten eine besonders große Anzahl an potentiellen Fehlerquellen korrigieren. Die Ermittlung der Kalibrationsdaten sollte dabei möglichst "spät" im Fertigungsprozess beziehungsweise Montageprozess der Teilchenenergieanpassvorrichtung erfolgen. Insbesondere ist es vorteilhaft, wenn die Messung zur Ermittlung der Kalibrationsdaten erst dann durchgeführt wird, wenn die Teilchenenergieanpassvorrichtung fertig im Zusammenhang mit der Teilchenbeschleunigervorrichtung und gegebenenfalls den Behandlungsplätzen und dergleichen erfolgt (mit anderen Worten unmittelbar bevor die Anlage den "Produktionsbetrieb" aufnehmen kann). In letzterem Fall ist insbesondere auch die Korrektur von Montagefehlern bei der Montage der Teilchenenergieanpassvorrichtung relativ zur Teilchenstrahlführung (zur Beampipe usw.) berücksichtigt. Ein weiteres bevorzugtes Merkmal ist es, wenn die Korrektur der Stellwerte unter Verwendung der Kalibrationsdaten in der Teilchenenergieanpassvorrichtung selbst erfolgt. Die Teilchenenergieanpassvorrichtung kann dann als "Blackbox" aufgefasst werden, die bei einer Ansteuerung mit Stellwerten eine besonders hochwertige und genaue Dämpfung zur Verfügung stellt. Dadurch kann die Teilchenenergieanpassvorrichtung als "Snap in"-Lösung verwendet werden. Dadurch werden insbesondere eine nachträgliche Montage und/oder ein Austausch einer Teilchenenergieanpassvorrichtung besonders einfach möglich. Insbesondere müssen die zur Korrektur erforderlichen Berechnungen nicht notwendigerweise auf den Computern der eigentlichen Beschleunigervorrichtung durchgeführt werden, sodass hier nicht notwendigerweise beispielsweise eine Anpassung der Rechenleistung, Eingriffe in den Programmablauf oder dergleichen durchgeführt werden müssen.

Grundsätzlich ist es möglich, dass die Kalibrationsdaten in beliebiger Art und Weise (genauer: in beliebiger geometrischer Lage) ermittelt werden. Es hat sich jedoch als vorteilhaft erwiesen, wenn die Kalibrationsdaten zumindest bereichsweise über eine Fläche hinweg ermittelt wurden, insbesondere in Form eines zweidimensionalen Gitters ermittelt wurden. Bei einer Vielzahl von Teilchenenergieanpassvorrichtungen ist es nämlich erforderlich, dass der Strahl nicht nur hinsichtlich seiner Energie angepasst wird, sondern auch hinsichtlich seiner lateralen Lage verändert werden kann. Insbesondere ist es die Regel, dass die laterale Lage in zwei Dimensionen verändert werden kann, der Strahl also somit vom Prinzip her beliebige Punkte innerhalb einer bestimmten Fläche (insbesondere Iso-Energieebene) "bestreichen" kann. Gemeinsam mit der Anpassung der Teilchenenergie (und damit der Lage der Iso-Energieebene in z-Richtung) kann somit ein an sich beliebiges dreidimensionales Volumen erreicht werden. Selbstverständlich ist die Größe und Ausdehnung der erreichbaren Fläche bzw. des erreichbaren Volumens durch die Teilchenbeschleunigervorrichtung (einschließlich ihrer Subsysteme, wie insbesondere auch der Teilchenenergieanpassvorrichtung) beschränkt. Eine Ermittlung von Kalibrationsdaten über eine Fläche hinweg erweist sich dabei insbesondere deshalb als besonders vorteilhaft, weil speziell dann, wenn der Teilchenstrahl in lateraler Richtung ausgelenkt wird, besonders große systematische Fehler auftreten können. Diese können beispielsweise durch die schräg verlaufende Durchtrittsbahn, aus geometrischen Gründen bzw. aufgrund von Parallaxenfehlern entstehen. Grundsätzlich ist es vorteilhaft, wenn die Ermittlung von Kalibrationsdaten in einem Bereich und insbesondere an solchen Punkten durchgeführt wird, an denen der Teilchenstrahl besonders häufig auf die Teilchenenergieanpassvorrichtung, insbesondere auf die Energievariationseinrichtung auftrifft. Insbesondere dann (aber nicht nur) wenn derartige Punkte nicht vorhanden oder (noch) nicht bekannt sind, kann zusätzlich oder alternativ auch eine Ermittlung in Form eines gegebenenfalls regelmäßigen zweidimensionalen Gitters erfolgen. Zusätzlich oder alternativ zur Verwendung eines (ansonsten regelmäßigen) Gitters ist es auch möglich, dass in Bereichen, in denen der Teilchenstrahl beispielsweise besonders häufig auf die Teilchenenergieanpassvorrichtung auftrifft, eine erhöhte Dichte an Messpunkten/Gitterpunkten verwendet wird. Auf diese Weise kann der gesamte Flächenbereich bei der Korrektur berücksichtigt werden (wenn auch gegebenenfalls nur näherungsweise).

Eine besonders bevorzugte Weiterbildung ergibt sich, wenn bei der Teilchenenergieanpassvorrichtung die Stellwertkorrektureinrichtung zumindest ein Interpolationsmittel aufweist. Mit einem derartigen Interpolationsmittel ist es möglich, dass eine (genauere) Korrektur auch in solchen Bereichen durchgeführt werden kann, in denen an sich kein Kalibrationsdatenpunkt (z. Bsp. ein Messwert) vorliegt. Die Zwischenwerte können beispielsweise durch lineare Interpolation, kubische Interpolation, Spline-Interpolation oder sonstige Interpolationsverfahren durchgeführt werden. Selbstverständlich ist es auch denkbar, dass anstelle einer Interpolation auch beispielsweise der Wert des nächstbenachbarten Kalibrationsdatenpunkts verwendet wird.

Weiterhin hat es sich als vorteilhaft erwiesen, wenn die Teilchenenergieanpassvorrichtung, insbesondere die Stellwertkorrektureinrichtung zumindest zeitweise und/oder zumindest bereichsweise eine Korrektur bezüglich der Veränderung der Energie der durch die Teilchenenergieanpassvorrichtung hindurch tretenden Teilchen durchführt. Dadurch kann die Energie der aus der Teilchenenergieanpassvorrichtung austretenden Teilchen besonders genau auf den gewünschten Wert eingestellt werden. Insbesondere die vorgeschlagene Korrektur in Bezug auf die Teilchenenergie erweist sich als besonders vorteilhaft, da die Teilchenenergieanpassvorrichtung in aller Regel die "letzte Instanz" bei der Anpassung der Teilchenenergie auf den gewünschten Wert darstellt. Eine (nochmalige) Anpassung der Teilchenenergie erfolgt somit in aller Regel nicht, sodass die Genauigkeit der Teilchenenergieanpassvorrichtung die Genauigkeit des gesamten Systems zumindest zu einem Großteil bestimmt.

Ebenso hat es sich als vorteilhaft erwiesen, wenn die Teilchenenergieanpassvorrichtung, insbesondere die Stellwertkorrektureinrichtung zumindest zeitweise und/oder zumindest bereichsweise eine Korrektur bezüglich der Flugbahn, insbesondere bezüglich der Flugrichtung und/oder bezüglich eines transversalen Versatzes der Teilchen durchführt. Speziell bei manchen Bauformen von Teilchenenergieanpassvorrichtungen (beispielsweise bei Bauformen mit keilartigem Energieabsorber bzw. doppelkeilartigem Energieabsorber) kann es aufgrund von physikalischen Effekten (speziell Beugungseffekten) zu einer Beeinflussung der Flugbahn der Teilchen kommen. Dementsprechend ist es von Vorteil, wenn dieser (typischerweise systematische) Fehler ebenfalls Berücksichtigung findet. Die Berücksichtigung kann darin bestehen, dass diese zu einer (zusätzlichen) Korrektur der Teilchenenergie führt. Die Korrektur kann aber auch darin bestehen, dass beispielsweise die Effekte auf die Teilchenflugbahn gemindert oder (im Wesentlichen) verhindert werden.

Eine weitere vorteilhafte Bauform der Teilchenenergieanpassvorrichtung ergibt sich, wenn die Teilchenenergieanpassvorrichtung, insbesondere die Stellwertkorrektureinrichtung zumindest eine vorzugsweise elektronische Recheneinrichtung und/oder zumindest eine vorzugsweise elektronische Speichereinrichtung aufweist. Als elektronische Recheneinrichtung und/oder als elektronische Speichereinrichtung können insbesondere numerisch-digitale Systeme verwendet werden. Hierbei ist nicht nur an übliche Computereinrichtungen zu denken, sondern beispielsweise auch an Einplatinen-Computer und dergleichen. Diese können insbesondere als eigenständige Einheit ausgebildet sein. Möglich ist es aber auch, dass die Funktionalität beispielsweise auf einer multitaskingfähigen Recheneinrichtung (die gegebenenfalls ohnehin vorhanden ist) quasi mit ausgeführt wird. Mit Hilfe der Recheneinrichtung ist es möglich, die Korrektur (insbesondere auch Interpolationen) besonders flexibel durchführen zu können. In der Speichereinrichtung können insbesondere die ermittelten Kalibrationsdaten gespeichert werden. Bei der Speichereinrichtung kann es sich in beliebiger Form um Speicherchips, aber auch um eine Festplatte oder dergleichen handeln. Vorzugsweise handelt es sich (zumindest teilweise) um nichtflüchtige Speicher, sodass die Daten auch bei einem Stromausfall nicht verloren gehen. Vorteilhaft ist es weiterhin, wenn die Teilchenenergieanpassvorrichtung, vorzugsweise die zumindest eine variable Energievariationseinrichtung zumindest eine Energieabsorptionseinrichtung aufweist, welche insbesondere zumindest teilweise und/oder zumindest bereichsweise als Verschiebekeileinrichtung, als schnell verfahrbare Wassersäuleneinrichtung und/oder als Modulatorradeinrichtung ausgebildet ist. Obgleich es grundsätzlich möglich ist, dass die Teilchenenergieanpassung nicht nur durch ein "Abbremsen", sondern auch durch ein "Beschleunigen" der Teilchen erfolgt, hat es sich als deutlich einfacher erwiesen, wenn in der Teilchenenergieanpassvorrichtung vorzugsweise bzw. lediglich ein Abbremsen der Teilchen stattfindet. Dieses ist nämlich in der Praxis deutlich einfacher zu handhaben und/oder zu bewerkstelligen. Eine gegebenenfalls zeitweise zu erhöhende Ausgangsenergie kann dadurch erreicht werden, das die Energie der die den Teilchenbeschleuniger verlassenden Teilchen geeignet hochgesetzt wird, um anschließend in der Teilchenenergieanpassvorrichtung auf den genauen, jeweils gewünschten Wert herabgesetzt zu werden. Die Erhöhung der Teilchenbeschleunigerleistung kann sowohl auf der Teilchenbeschleunigerseite erfolgen (wobei die Teilchenbeschleunigerseite nach einer Erhöhung ihrer Teilchenenergie das an die Teilchenenergieanpassvorrichtung gesendete Stellwertsignal entsprechend anpasst). Zusätzlich oder alternativ ist es jedoch auch möglich, dass die Teilchenenergieanpassvorrichtung ein entsprechendes Rückkopplungssignal an die Teilchenbeschleunigereinrichtung sendet, wenn es den jeweils aktuell gewünschten Wert nicht (mehr) bereitstellen kann. Dies kann sich im Übrigen nicht nur auf die Erhöhung der Teilchenbeschleunigerleistung, sondern auch sinngemäß auf eine Verringerung der Teilchenbeschleunigerleistung beziehen. Als bevorzugte Bauformen für die Energieabsorptionseinrichtung haben sich insbesondere Verschiebekeileinrichtungen, schnell verfahrbare Wassersäuleneinrichtungen und/oder Modulatorradeinrichtungen bewährt. Dies gilt insbesondere für Verschiebekeileinrichtungen. Verschiebekeileinrichtungen können dabei nicht nur als einzelne Verschiebekeile, sondern (bevorzugt) als Doppelkeilsysteme ausgeführt werden. Auch das Vorsehen einer größeren (vorzugsweise geradzahligen) Anzahl von Keilen ist möglich. Das Vorsehen einer geradzahligen Anzahl von Keilen ist dabei besonders vorteilhaft, da hier aus geometrischen Gründen Fehler die durch eine laterale Auslenkung des Teilchenstrahls entstehen können "hardwareseitig" minimiert werden können. Dieser Vorteil gleicht in aller Regel den Nachteil aus, der durch die üblicherweise komplexere Bauform entsteht aus und überkompensiert diesen üblicherweise.

Weiterhin wird eine Stellwertkorrektureinrichtung für eine Teilchenenergieanpassvorrichtung zur variablen Veränderung der Energie der Teilchen eines durch die Teilchenenergieanpassvorrichtung hindurch tretenden Teilchenstrahls vorgeschlagen, wobei die Stellwertkorrektureinrichtung derart ausgebildet und eingerichtet ist, dass die der Stellwertkorrektureinrichtung zugeführten Stellwerte zumindest zeitweise und/oder zumindest teilweise unter Verwendung von Kalibrationsdaten korrigiert werden. Dabei ist es möglich, die Stellwertkorrektureinrichtung im Sinne der obigen Beschreibung auszubilden und/oder weiterzubilden. Eine derartige Stellwertkorrektureinrichtung ist insbesondere besonders vorteilhaft zur Verwendung in einer der oben beschriebenen Teilchenenergieanpassvorrichtungen. Bei entsprechender Aus- bzw. Weiterbildung der Stellwertkorrektureinrichtung weist diese darüber hinaus die oben beschriebenen Vorteile und Eigenschaften in analoger Weise auf.

Weiterhin wird ein Verfahren zur Bestimmung von Korrekturwerten, insbesondere zur Verwendung für eine Teilchenenergieanpassvorrichtung, für eine Stellwertkorrektureinrichtung einer Teilchenenergieanpassvorrichtung bzw. für eine Stellwertkorrektureinrichtung mit dem oben beschriebenen Aufbau vorgeschlagen, bei dem zumindest ein Kalibrationswert, vorzugsweise eine Mehrzahl von Kalibrationsdaten ermittelt wird. Die Ermittlung kann - wie oben bereits sinngemäß erläutert- sowohl rechnerisch, als auch aufgrund von Messungen durchgeführt werden. Bevorzugt ist dabei die Verwendung von Messwerten. Ebenso ist es bevorzugt, dass die Messungen möglichst "spät" durchgeführt werden, sodass bei den Kalibrationswerten eine möglichst große Anzahl von (systematischen) Fehlerquellen berücksichtigt werden kann.

Das vorgeschlagene Verfahren kann im Sinne der obigen Beschreibung ausgebildet und insbesondere auch weitergebildet werden. Das vorgeschlagene Verfahren weist dann die bereits vorab beschriebenen Vorteile und Eigenschaften in analoger Weise auf.

Insbesondere ist es möglich, das Verfahren derart fortzubilden, dass die Kalibrationsdaten für eine flächige Anordnung, insbesondere für eine flächige Gitteranordnung ermittelt werden. Die besonderen Vorteile und Eigenschaften, die hierdurch erzielt werden können, wurden ebenfalls (zumindest sinngemäß) bereits vorab beschrieben.

Eine weitere besonders bevorzugte Weiterbildung des Verfahrens besteht darin, dass die Daten vor einer Verwendung der Stellwertkorrektureinrichtung und/oder vor einer Verwendung der Teilchenenergieanpassvorrichtung gewonnen und/oder in einer vorzugsweise elektronischen Speichereinrichtung gespeichert werden. Wie bereits erläutert, sollte die Gewinnung der Daten möglichst "spät" erfolgen, damit möglichst viele potentielle Fehlerquellen berücksichtigt werden können. Ansonsten wird (zumindest sinngemäß) auf die bereits angegebene Beschreibung von Ausbildungsmöglichkeiten, Weiterbildungsmöglichkeiten, Vorteilen und Eigenschaften verwiesen.

Im Folgenden wird die Erfindung anhand vorteilhafter Ausführungsbeispiele und unter Bezugnahme auf die beigefügte Zeichnung näher erläutert. Es zeigen:
- Fig. 1:: einen Doppelkeil-Energieabsorber für einen Teilchenstrahl in einer schematischen Prinzipdarstellung;
- Fig. 2:: die unterschiedlichen Maximalpositionen des in Fig. 1 dargestellten Energiemodulators in einer schematischen Draufsicht;
- Fig. 3:: eine schematische Darstellung eines Messpunktrasters zur Ermittlung von Kalibrationsdaten;
- Fig. 4:: einen schematischen Ablaufplan eines Teilchenenergieanpassverfahrens.

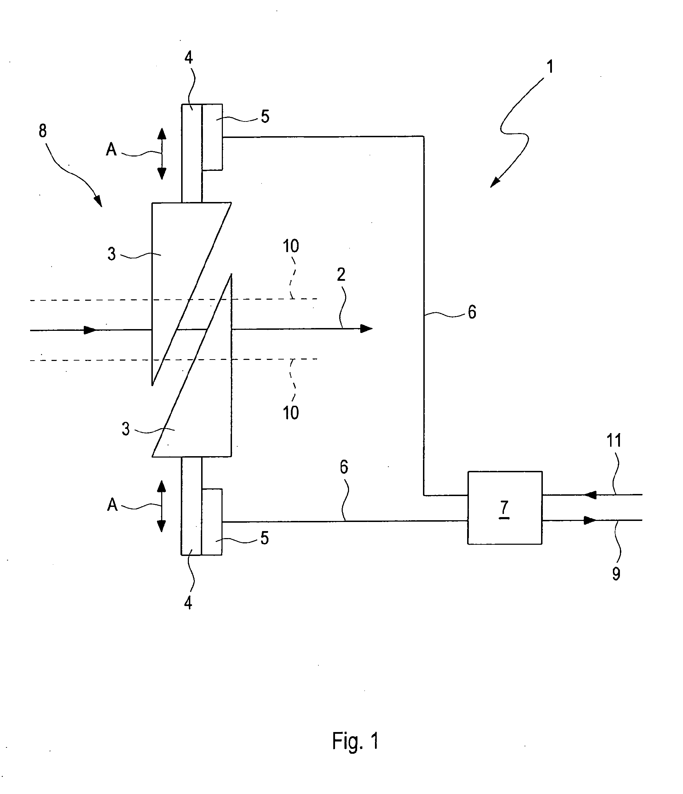

In Fig. 1 ist in einer schematischen, perspektivischen Prinzipdarstellung ein Energiemodulator 1 mit seinen wesentlichen Baugruppen dargestellt. Der Energiemodulator 1 dient der unterschiedlich starken Dämpfung (Energieabsorption; Verlangsamung) eines durch den Energiemodulator 1 hindurch tretenden Teilchenstrahls 2. Die eigentliche Dämpfung des Teilchenstrahls 2 erfolgt in den vorliegend zwei punktsymmetrisch zueinander angeordneten Keilen 3. Die beiden Keile 3 sind aus einem energieabsorbierenden Material mit einer möglichst guten Materialhomogenität gefertigt. Dennoch lässt es sich in der Praxis nie vermeiden, dass es bei der Fertigung der Keile 3 zu Materialinhomogenitäten und/oder zu Inhomogenitäten der Oberfläche (Forminhomogenitäten) kommt. Dadurch kommt es zu (initial) eratischen Schwankungen bei der Dämpfung des durch den Energiemodulator 1 hindurch tretenden Teilchenstrahls 2. Ein typisches Material für die Keile 3 ist Plexiglas. Grundsätzlich können jedoch auch andere Materialien hierzu verwendet werden.

Die beiden Keile 3 sind jeweils an Haltestangen 4 befestigt und sind mit Hilfe von Linearmotoren 5 relativ zueinander verschiebbar (in Fig. 1 jeweils durch einen Doppelpfeil A angedeutet). Die Linearmotoren 5 werden über Steuerkabel 6 von einem vorliegend als Einplatinen-Computer ausgebildeten elektronischen Rechner 7 angesteuert. Die Ansteuerung erfolgt dabei derart, dass die beiden Keile 3 jeweils in unterschiedlichen Richtungen gleichartig zueinander aufeinander zu bewegt bzw. voneinander weg bewegt werden. Je nach Stellung der beiden Keile 3 relativ zueinander legt der Teilchenstrahl 2 (wie aus Fig. 1 leicht ersichtlich ist) eine unterschiedlich weite Wegstrecke durch das Material der beiden Keile hindurch zurück. Da die Energiedämpfung mit der im Material der Keile 3 zurückgelegten Wegstrecke korreliert erfährt der Teilchenstrahl 2 zwischen Eintritt in den Energiemodulator 1 und Austritt aus dem Energiemodulator 1 eine unterschiedlich starke Dämpfung. Die Baugruppe aus den beiden Keilen 3 wirkt somit als reine Energiedämpfungseinheit 8, deren Dämpfungswirkung jedoch mit Hilfe der Linearmotoren 5 verändert werden kann. Eine Beschleunigung des Teilchenstrahls 2 ist bei der vorliegend dargestellten Bauausführung des Energiemodulators 1 nicht möglich. Sollte eine Energieerhöhung über den maximal möglichen Ausgangswert des Energiemodulators 1 (minimale Dämpfungswirkung der Energiedämpfungseinheit 8) hinaus erforderlich sein, so kann der elektronische Rechner 7 über eine Datenleitung 9 ein Signal an den dem Energiemodulator 1 vorgeschalteten Teilchenbeschleuniger (nicht dargestellt) senden, dass dieser die Teilchenenergie in geeignetem Maße erhöht. Entsprechendes kann auch gelten, wenn die gewünschte Teilchenenergie auf ein Niveau abgesenkt werden soll, das unterhalb der minimalen Ausgangsenergie des Energiemodulators 1 (maximale Dämpfungswirkung der Energiedämpfungseinheit 8) liegt.

Aufgrund der symmetrischen Ausbildung und Anordnung der Keile 3 verändert sich die Dämpfungswirkung des Doppelkeilsystems (der Energiedämpfungseinheit 8) nicht, wenn der Teilchenstrahl 3 seitlich versetzt in den Energiemodulator 1 eintritt (in Fig. 1 sind zwei lateral versetzte Teilchenstrahle 10 eingezeichnet). Dies rührt daher, dass beim lateral versetzten Teilchenstrahl 10 die Wegstrecke, die der lateral versetzte Teilchenstrahl 10 beispielsweise im vorderen Keil 3 zurücklegt, eine entsprechend verkürzte Wegstrecke im hinten liegenden Keil 3 zur Folge hat (und umgekehrt). Selbstverständlich ist hierdurch nicht ausgeschlossen, dass Effekte höherer Ordnung eine (üblicherweise kleinere) Veränderung der Dämpfungswirkung des Teilchenstrahls 3, 10 zur Folge haben können.

Die Ansteuerung des Energiemodulators 1 erfolgt über eine Datenleitung 11, die in den elektronischen Rechner 7 führt. Über die Datenleitung 11 können (unkorrigierte) Stellwerte eingegeben werden, wie beispielsweise eine gewünschte Dämpfungswirkung der Energiedämpfungseinheit 8. Diese Stellwerte können beispielsweise von einem Zentralrechner des Teilchenbeschleunigers, der den Teilchenstrahl 2, 10 erzeugt, vorgegeben werden. Die Eingabe über die Datenleitung 11 ist jedoch nicht notwendigerweise hierauf beschränkt. Beispielsweise können über die Datenleitung 11 zusätzliche Informationen, wie beispielsweise das Ausmaß eines lateralen Versatzes eines lateral versetzten Teilchenstrahls 10 mit eingegeben werden. Mit solchen Daten ist es mit dem elektronischen Rechner 7 möglich, eine bessere Korrektur der Dämpfungswirkung der Energiedämpfungseinheit 8 durchzuführen (im Folgenden beschrieben). Die eingegebenen Daten über den lateralen Versatz müssen dabei nicht notwendigerweise Messwerte sein, sondern können beispielsweise auch die Stellwerte sein, die an eine Einheit übergeben werden, die den lateralen Versatz des Teilchenstrahls 2, 10 bewirkt. Als derartige laterale Versatzeinheit können beispielsweise zwei zueinander senkrecht angeordnete (und jeweils senkrecht zur Teilchenstrahlrichtung stehende) Magnetspulenpaare verwendet werden (vorliegend nicht dargestellt). Lediglich der Vollständigkeit halber sollte darauf hingewiesen werden, dass die Datenleitung 11 insbesondere als paketorientierte Datenleitung (beispielsweise Ethernet-Protokoll, Token-Ring-Protokoll, Glasfaserdatenkabel usw.) ausgeführt sein kann. Auch ist es möglich, dass insbesondere bei einer solchen "paketorientierten" Bauausführung die Datenleitung 11 für das Eingabesignal und die Datenleitung 9 für das Rückkopplungssignal als gemeinsame Datenleitung ausgeführt sein können (vorliegend nicht dargestellt).

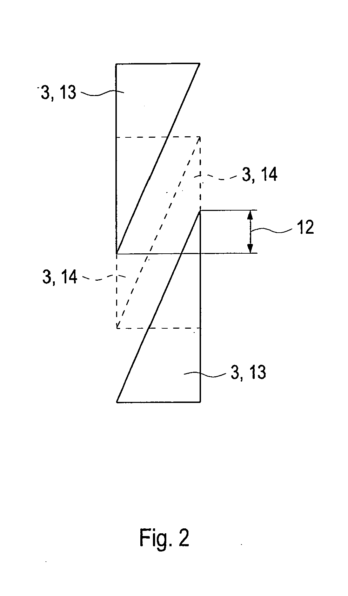

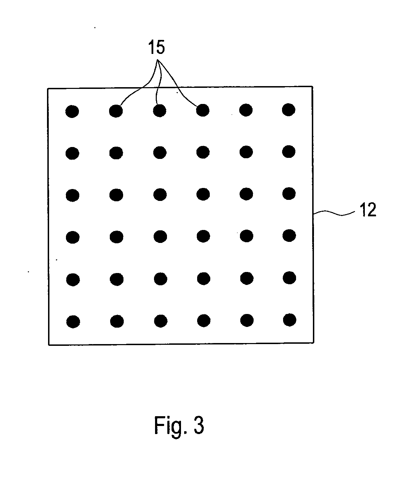

In Fig. 2 sind die beiden Keile 3 des in Fig. 1 dargestellten Energiemodulators 1 jeweils in einer maximal zueinander beabstandeten Stellung 13, sowie in einer minimal zueinander beabstandeten Stellung 14 (mit einer gestrichelten Linie eingezeichnet) dargestellt. Der zur Dämpfung des Teilchenstrahls 2, 10 nutzbare Bereich 12 ist dabei durch den Überlappungsbereich der beiden Keile bei der maximal zueinander beabstandeten Stellung 13 definiert. Dieser nutzbare Bereich 12 ist in Fig. 3 in Draufsicht dargestellt.

In Fig. 3 ist innerhalb des nutzbaren Bereichs 12 eine Vielzahl von Messpunkten 15 dargestellt. Die Messpunkte 15 sind im vorliegend dargestellten Ausführungsbeispiel in Form eines regelmäßigen Gitters angeordnet. Der Abstand zweier Messpunkte 15 ist vorliegend entlang einer Zeile, als auch entlang einer Spalte jeweils konstant. Es sind jedoch grundsätzlich auch andere Muster möglich. Insbesondere kann eine Häufung von Punkten in einem Bereich vorgenommen werden, in dem der Teilchenstrahl 2, 10 typischerweise bzw. besonders häufig auftrifft.

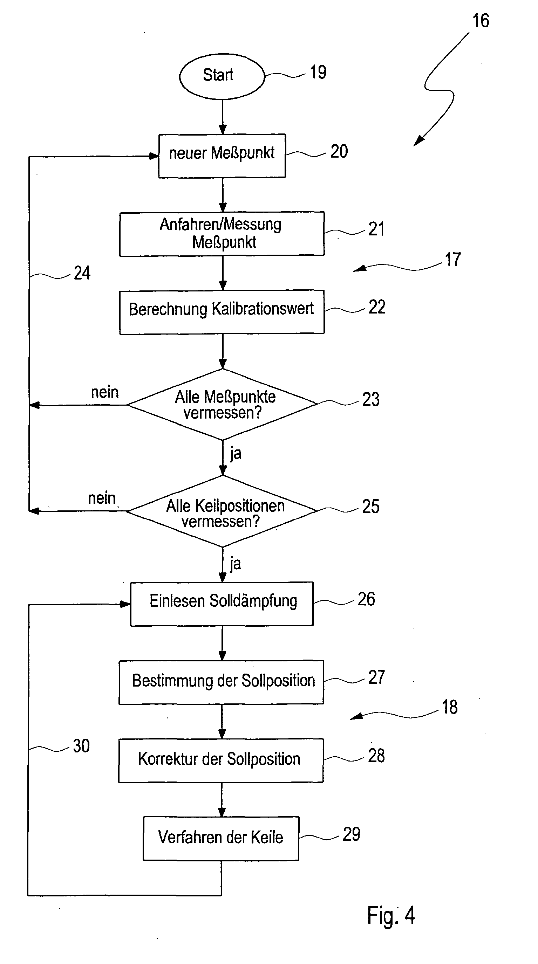

Die einzelnen Messpunkte 15 werden, beispielsweise nachdem der Energiemodulator 1 in die Teilchenbeschleunigervorrichtung eingebaut wurde, nacheinander angefahren (vergleiche auch Fig. 4). Durch Messung wird für jeden der einzelnen Messpunkte 15 die tatsächliche Dämpfungswirkung experimentell bestimmt. Der Unterschied zwischen tatsächlicher und "theoretischer" Dämpfung wird einzeln für jeden Messpunkt 15 berechnet und in einer Speichereinheit des elektronischen Rechners 7 abgelegt (beispielsweise als so genannte "look up"-Tabelle). Diese Werte werden anschließend in einem "Produktionsbetrieb" des Teilchenbeschleunigers bzw. des Energiemodulators 1 als Kalibrationsdaten verwendet.

Die Messung der tatsächlichen Dämpfung pro Messpunkt 15 wird dabei nicht nur in einer einzelnen Stellung der beiden Keile 3 zueinander durchgeführt, sondern sowohl bei maximaler Entfernung 13 und minimaler Entfernung 14 der beiden Keile 3 zueinander, als auch bei einer geeignet großen Anzahl von Zwischenstellungen.

Die Dichte des Punktrasters 15 sowie die Anzahl der Zwischenstellungen der beiden Keile 3 zueinander sollte einerseits ausreichend groß gewählt werden, sodass eine ausreichend gute Kalibration möglich ist, andererseits aber auch hinreichend klein gewählt werden, damit die Vermessung nicht übermäßig lang dauert. Sollte im "Produktionsbetrieb" ein Wert nachgefragt werden, der nicht vermessen wurde, so kann beispielsweise der Wert des zunächst benachbarten Messpunkts 15 verwendet werden. Möglich ist es jedoch auch, dass ein Wert unter Verwendung von Interpolationsverfahren mit den benachbarten Messpunkten 15 ermittelt wird.

In Fig. 4 ist das Verfahren 16, dass für "Bau" und Betrieb eines Energiemodulators (beispielsweise des in Fig. 1 gezeigten Energiemodulators 1) verwendet werden kann, dargestellt. Das Gesamtverfahren 16 gliedert sich im Wesentlichen zwei Teilverfahren 17, 18, nämlich das Verfahren zur Ermittlung von Kalibrationsdaten 17, sowie das Verfahren zur Korrektur von Stellwerten 18. Möglich ist es dabei insbesondere, dass das Verfahren 17 nur ein einziges Mal durchgeführt wird, und die dabei ermittelten Kalibrationsdaten beispielsweise in einem nicht-flüchtigen Speicher eines elektronischen Rechners 7 abgelegt werden. Es ist jedoch auch möglich, dass das Verfahren zur Ermittlung von Kalibrationsdaten 17 in periodischen Abständen durchgeführt wird. Beispielsweise ist es möglich, dass das Verfahren zur Ermittlung von Kalibrationsdaten 17 beispielsweise jeweils am Anfang eines Therapietages durchgeführt wird, um jeweils aktuelle Korrekturdaten vorliegen zu haben.

Das Gesamtverfahren 16 startet mit dem Startschritt 19. Hier wird beispielsweise der elektronische Rechner 7 initialisiert und dergleichen.

In einem ersten Verfahrensschritt 20 wird ein erster (bzw. ein neuer) Messpunkt 15 ermittelt, der zur Messung der tatsächlichen Dämpfungswirkung der Energiedämpfereinheit 8 bzw. des Energiemodulators 1 angefahren werden soll. Der derart ermittelte 20 neue Messpunkt 15 wird anschließend angefahren 21. Ein entsprechendes Signal kann beispielsweise über die Rückkopplungs-Datenleitung 9 ausgegeben werden. Darüber hinaus wird im Verfahrensschritt 21 die tatsächliche Dämpfungswirkung gemessen.

Sobald die Ergebnisse vorliegen wird aus dem in Schritt 21 gewonnenen Daten der für den aktuellen Messpunkt 15 gültige Kalibrationswert berechnet 22.

Somit ist die Vermessung des ersten Messpunkts 15 abgeschlossen. Im Anschluss danach wird in einem Überprüfungsschritt 23 überprüft, ob sämtliche Messpunkte 15 des Messgitters bereits vermessen wurden. Ist dies nicht der Fall, so springt das Verfahren zurück 24 zum Schritt 20, wo ein neuer Messpunkt 15 bestimmt wird. Ist das Raster dagegen vollständig vermessen, so wird in einem nachgeschalteten Überprüfungsschritt 25 überprüft, ob alle erwünschten Positionen der Keile 3 relativ zueinander vermessen wurden. Ist dies nicht der Fall, so werden die beiden Keile 3 in eine neue Position relativ zueinander verfahren und das Verfahren springt zurück 24 zum Verfahrensschritt 20 wo ein neuer (erster) Messpunkt 15 ermittelt wird 20, der anschließend vermessen wird.

Sind dagegen alle Keilpositionen vermessen worden, so wird das Verfahren zur Ermittlung der Kalibrationsdaten 17 verlassen und das Verfahren zur Ermittlung korrigierter Stellwerte 18 begonnen. Hierbei wird jeweils über eine Datenleitung 11 ein erwünschter Dämpfungswert eingelesen 26, der von der Energiedämpfungseinrichtung 8 bzw. dem Energiemodulator 1 eingenommen werden soll. Die Daten werden beispielsweise vom Hauptrechner einer Teilchenbeschleunigeranlage zur Verfügung gestellt. Aus diesem Sollwert wird in einem nächsten Schritt 27 die Sollposition der Keile 3 relativ zueinander in "nullter Näherung" ermittelt. Dies kann beispielsweise durch analytische Verfahren erfolgen.

Die derart ermittelten Sollwerte werden in einem nachgeschalteten Verfahrensschritt 28 korrigiert. Hierbei werden die im ersten Verfahrensblock 17 gewonnenen Kalibrationsdaten verwendet. Durch die Korrektur der Stellwerte können beispielsweise Inhomogenitäten bezüglich der Oberfläche der Keile 3, bezüglich des Materials der Keile 3 (z. Bsp. unterschiedliche Materialdichten), Stellwertfehler der Linearmotoren 5 und dergleichen berücksichtigt werden. Die Korrektur der Stellwerte 28 kann eine erhöhte Genauigkeit der tatsächlichen Dämpfungswirkung des Energiemodulators 1 bzw. der Energiedämpfungseinheit 8 bewirken.

In einem anschließenden Verfahrensschritt 29 werden die derart gewonnenen korrigierten Positionssollwerte umgesetzt, das heißt die Keile 3 werden in die entsprechende, korrigierte Sollposition verfahren 29. Anschließend springt das Verfahren zurück 30 zum Verfahrensschritt 26, wo ein neuer Sollwert eingelesen wird.

**Bezugszeichenliste:**

| | | | |
|---|---|---|---|
| 1. | Energiemodulator | 18. | Verfahren zur Korrektur von Stellwerten |
| 2. | Teilchenstrahl | | |
| 3. | Keil | 19. | Startpunkt |
| 4. | Haltestange | 20. | Ermittlung neuer Mess-punkt |
| 5. | Linearmotor | | |
| 6. | Steuerkabel | 21. | Anfahren/Messung Mess-punkt |
| 7. | Elektronischer Rechner | | |
| 8. | Energiedämpfungseinheit | 22. | Berechnung Kalibrationswert |
| 9. | Datenleitung (Rückkopplung) | | |
| | | 23. | Überprüfung Ende Mess-raster |
| 10. | Lateral verschobener Teilchenstrahl | | |
| | | 24. | Rücksprung |
| 11. | Datenleitung (Eingabe) | 25. | Überprüfung Keilposition |
| 12. | Nutzbarer Bereich | 26. | Einlesen Solldämpfung |
| 13. | Maximale Entfernung | 27. | Bestimmung Sollposition |
| 14. | Minimale Entfernung | 28. | Korrektur der Sollposition |
| 15. | Messpunkt | 29. | Verfahren der Keile |
| 16. | Gesamtverfahren | 30. | Rücksprung |
| 17. | Verfahren zur Ermittlung von Kalibrationsdaten | | |

## Patentansprüche

1. Teilchenenergieanpassvorrichtung (1) zur variablen Veränderung der Energie der Teilchen eines durch die Teilchenenergieanpassvorrichtung (1) hindurch tretenden Teilchenstrahls (2, 10), aufweisend zumindest eine variable Energievariationseinrichtung (8), **gekennzeichnet durch** zumindest eine Stellwertkorrektureinrichtung (7) zur Korrektur eines der Teilchenenergieanpassvorrichtung (1) zugeführten Stellwerts (11), wobei die Stellwertkorrektureinrichtung (7) derart ausgebildet und eingerichtet ist, dass die der Teilchenenergieanpassvorrichtung (1) zugeführten Stellwerte (11) zumindest zeitweise und/oder zumindest teilweise unter Verwendung von Kalibrationsdaten (15) korrigiert werden und wobei Kalibrationsdaten (15) zumindest bereichsweise über eine Fläche hinweg ermittelt werden, insbesondere in Form eines zweidimensionalen Gitters (Fig. 3) ermittelt werden.

2. Teilchenenergieanpassvorrichtung (1) nach Anspruch 1, **dadurch gekennzeichnet, dass** die Stellwertkorrektureinrichtung (7) zumindest ein Interpolationsmittel aufweist.

3. Teilchenenergieanpassvorrichtung (1) nach einem der vorangehenden Ansprüche, **dadurch gekennzeichnet, dass** die Teilchenenergieanpassvorrichtung (1), insbesondere die Stellwertkorrektureinrichtung (7) zumindest zeitweise und/oder zumindest bereichsweise eine Korrektur bezüglich der Veränderung der Energie der durch die Teilchenenergieanpassvorrichtung hindurch tretenden Teilchen (2, 10) durchführt.

4. Teilchenenergieanpassvorrichtung (1) nach einem der vorangehenden Ansprüche, **dadurch gekennzeichnet, dass** die Teilchenenergieanpassvorrichtung (1), insbesondere die Stellwertkorrektureinrichtung (7) zumindest zeitweise und/oder zumindest bereichsweise eine Korrektur bezüglich der Flugbahn, insbesondere bezüglich der Flugrichtung und/oder bezüglich eines transversalen Versatzes (10) der Teilchen durchführt.

5. Teilchenenergieanpassvorrichtung (1) nach einem der vorangehenden Ansprüche, **dadurch gekennzeichnet, dass** die Teilchenenergieanpassvorrichtung (1), insbesondere die Stellwertkorrektureinrichtung (7) zumindest eine vorzugsweise elektronische Recheneinrichtung (7) und/oder zumindest eine vorzugsweise elektronische Speichereinrichtung aufweist.

6. Teilchenenergieanpassvorrichtung (1) nach einem der vorangehenden Ansprüche, **dadurch gekennzeichnet, dass** die Teilchenenergieanpassvorrichtung (1), vorzugsweise die zumindest eine variable Energievariationseinrichtung (8) zumindest eine Energieabsorptionseinrichtung (8) aufweist, welche insbesondere zumindest teilweise und/oder zumindest bereichsweise als Verschiebekeileinrichtung (8), als schnell verfahrbare Wassersäuleneinrichtung und/oder als Modulatorradeinrichtung ausgebildet ist.

7. Verfahren (16, 17) zur Bestimmung von Korrekturwerten, insbesondere von Korrekturwerten zur Verwendung für eine Teilchenenergieanpassvorrichtung (1) nach einem der Ansprüche 1 bis 6, für eine Stellwertkorrektureinrichtung (7) einer Teilchenenergieanpassvorrichtung (1) nach einem der Ansprüche 1 bis 6, **dadurch gekennzeichnet, dass** zumindest ein Kalibrationswert (15), insbesondere eine Mehrzahl von Kalibrationsdaten (15) ermittelt wird und dass die Kalibrationsdaten (15) für eine flächige Anordnung, insbesondere für eine flächige Gitteranordnung (Fig. 3), ermittelt werden.

8. Verfahren (16, 17) nach Anspruch 7, **dadurch gekennzeichnet, dass** die Kalibrationsdaten vor einer Verwendung der Stellwertkorrektureinrichtung (7) und/oder vor einer Verwendung der Teilchenenergieanpassvorrichtung (1) gewonnen und/oder in einer vorzugsweise elektronischen Speichereinrichtung gespeichert werden.

## Claims

1. A particle energy modulating device (1) for variably changing the energy of particles of a particle beam (2, 10) passing through the particle energy modulating device (1), comprising at least one variable energy varying device (8), **characterized by** at least one control value correcting device (7) for correcting a control value (11) that is supplied to the particle energy modulating device (1), wherein the control value correcting device (7) is configured and adapted to correct the control values (11) supplied to the particle energy modulating device (1) at least sometimes and/or at least partially using calibration data (15), and wherein in at least some regions calibration data (15) are determined over an area, in particular in form of a two-dimensional grid (Fig. 3).

2. The particle energy modulating device (1) according to claim 1, **characterized in that** the control value correcting device (7) includes at least one interpolation means.

3. The particle energy modulating device (1) according to any of the preceding claims, **characterized in that** the particle energy modulating device (1), in particular the control value correcting device (7), performs a correction with regard to the change in energy of the particles (2, 10) passing through the particle energy modulating device, at least sometimes and/or in at least some regions.

4. The particle energy modulating device (1) according to any of the preceding claims, **characterized in that** the particle energy modulating device (1), in particular the control value correcting device (7), performs a correction with regard to the trajectory of the particles, in particular with regard to a flight direction and/or with regard to a transverse offset (10) thereof, at least sometimes and/or in at least some regions.

5. The particle energy modulating device (1) according to any of the preceding claims, **characterized in that** the particle energy modulating device (1), in particular the control value correcting device (7), includes at least one preferably electronic computing means (7) and/or at least one preferably electronic memory means.

6. The particle energy modulating device (1) according to any of the preceding claims, **characterized in that** the particle energy modulating device (1), preferably the at least one variable energy varying device (8), includes at least one energy absorption means (8) which is in particular configured as a sliding wedge means (8), as a rapidly displaceable water column means, and/or as a modulator wheel means, at least partially and/or in at least some regions.

7. A method (16, 17) for determining correction values, in particular correction values for use in a particle energy modulating device according to any of claims 1 to 6, for a control value correcting device (7) of a particle energy modulating device (1) according to any of claims 1 to 6, **characterized in that** at least one calibration value (15) is determined, in particular a plurality of calibration data (15), and **in that** the calibration data (15) are determined for a two-dimensional array, in particular for a two-dimensional grid array (Fig. 3).

8. The method (16, 17) according to claim 7, **characterized in that** the calibration data are acquired before the control value correcting device (7) is used and/or before the particle energy modulating device (1) is used and/or are stored in a preferably electronic memory me

## Revendications

1. Dispositif d'adaptation de l'énergie des particules (1) pour une modification variable de l'énergie des particules d'un faisceau de particules (2, 10) traversant le dispositif d'adaptation de l'énergie des particules (1), présentant au moins un système de variation d'énergie (8) variable, **caractérisé en ce qu'**il comporte au moins un système de correction de valeur de réglage (7) pour la correction d'une valeur de réglage (11) acheminée au dispositif d'adaptation de l'énergie des particules (1), le système de correction de valeur de réglage (7) étant conçu et agencé de manière telle que les valeurs de réglage (11) acheminées au dispositif d'adaptation de l'énergie des particules (1) soient corrigées au moins temporairement et/ou au moins partiellement en utilisant des données d'étalonnage (15), et des données d'étalonnage (15) étant déterminées au moins dans certaines zones en travers d'une surface et étant déterminées notamment sous la forme d'un réseau bidimensionnel (fig. 3).

2. Dispositif d'adaptation de l'énergie des particules (1) selon la revendication 1, **caractérisé en ce que** le système de correction de valeur de réglage (7) présente au moins un moyen d'interpolation.

3. Dispositif d'adaptation de l'énergie des particules (1) selon l'une des revendications précédentes, **caractérisé en ce que** le dispositif d'adaptation de l'énergie des particules (1), notamment le système de correction de valeur de réglage (7), effectue au moins temporairement et/ou au moins dans certaines zones une correction par rapport à la modification de l'énergie des particules (2, 10) traversant le dispositif d'adaptation de l'énergie des particules.

4. Dispositif d'adaptation de l'énergie des particules (1) selon l'une des revendications précédentes, **caractérisé en ce que** le dispositif d'adaptation de l'énergie des particules (1), notamment le système de correction de valeur de réglage (7), effectue au moins temporairement et/ou au moins dans certaines zones une correction par rapport à la trajectoire de vol, en particulier par rapport à la direction de vol, et/ou par rapport à un décalage transversal (10) des particules.

5. Dispositif d'adaptation de l'énergie des particules (1) selon l'une des revendications précédentes, **caractérisé en ce que** le dispositif d'adaptation de l'énergie des particules (1), notamment le système de correction de valeur de réglage (7), présente au moins un système de calcul (7), de préférence électronique, et/ou au moins un système de mémoire, de préférence électronique.

6. Dispositif d'adaptation de l'énergie des particules (1) selon l'une des revendications précédentes, **caractérisé en ce que** le dispositif d'adaptation de l'énergie des particules (1), de préférence le système de variation d'énergie (8) variable, au nombre d'au moins un, présente au moins un système d'absorption d'énergie (8) qui est réalisé notamment au moins en partie et/ou au moins dans certaines zones en tant que système à coin coulissant (8), en tant que système à colonne d'eau à déplacement rapide et/ou en tant que système à roue de modulateur.

7. Procédé (16, 17) pour déterminer des valeurs de correction, en particulier des valeurs de correction destinées à être utilisées pour un dispositif d'adaptation de l'énergie des particules (1) selon l'une des revendications 1 à 6, pour un système de correction de valeur de réglage (7) d'un dispositif d'adaptation d'énergie des particules (1) selon l'une des revendications 1 à 6, **caractérisé en ce que** l'on détermine au moins une valeur d'étalonnage (15), notamment une pluralité de données d'étalonnage (15), et **en ce que** les données d'étalonnage (15) sont déterminées pour un agencement plan, en particulier pour un agencement en réseau plan (fig. 3).

8. Procédé (16, 17) selon la revendication 7, **caractérisé en ce que**, avant une utilisation du système de correction de valeur de réglage (7) et/ou avant une utilisation du dispositif d'adaptation de l'énergie des particules (1), les données d'étalonnage sont obtenues et/ou sont enregistrées dans un système de mémoire, de préférence électronique.
